(19) Europäisches Patentamt
European Patent Office
Office européen des brevets

(11) EP 3 338 860 A1

(12) EUROPEAN PATENT APPLICATION

(43) Date of publication:
27.06.2018 Bulletin 2018/26

(51) Int Cl.:
A61N 5/10 (2006.01)

(21) Application number: 16206519.7

(22) Date of filing: 22.12.2016

(84) Designated Contracting States:
AL AT BE BG CH CY CZ DE DK EE ES FI FR GB
GR HR HU IE IS IT LI LT LU LV MC MK MT NL NO
PL PT RO RS SE SI SK SM TR
Designated Extension States:
BA ME
Designated Validation States:
MA MD

(71) Applicants:
• Ion Beam Applications S.A.
1348 Louvain-la-Neuve (BE)

• Université catholique de Louvain
1348 Louvain-la-Neuve (BE)

(72) Inventors:
• Vander Stappen, François Michel P.
B-1348 Louvain-la-Neuve (BE)
• Deffet, Sylvain Jean G.
1348 Louvain-la-Neuve (BE)

(74) Representative: Nederlandsch Octrooibureau
P.O. Box 29720
2502 LS The Hague (NL)

(54) REGISTRATION OF PARTICLE BEAM RADIOGRAPHY DATA

(57) An apparatus for aligning a volumetric dataset to a particle beam radiography data is disclosed. The apparatus comprises a particle source (101) for applying a particle beam directed toward an object, wherein the particle source is configured to scan at least part of the object. The apparatus comprises a particle detector (102) for measuring integral depth dose distributions of the respective pencil beam shots downstream of the object. The apparatus comprises a controller (104) configured to calculate reference depth dose distributions corresponding to the respective pencil beam shots based on the information about the stopping power and an assumed position of the object as represented by the volumetric dataset relative to the respective pencil beam shots, compare the measured depth dose distributions to their corresponding reference depth dose distributions, and compute the position of the object relative to the pencil beam shots based on an outcome of the comparison.

Fig. 5

EP 3 338 860 A1

**Description**

**Field of the invention**

The present invention relates to registration of particle beam radiography data. More particularly, the present invention relates to registration of particle radiography measurements to a volumetric dataset, such as a CT dataset or an MRI dataset.

**Background art**

Charged particles (i.e. protons, ions such as carbon ions) have physical advantages with respect to X-rays or gamma rays in the field of radiation therapy. For example, protons of a given energy (i.e. forming a mono-energetic proton beam), have a certain penetration depth in a target object and do not penetrate beyond that depth, and furthermore, they deposit their maximum amount of energy or dose in the so-called Bragg Peak, which corresponds to said penetration depth, i.e. the point of greatest penetration of the radiation in the target volume. The position of the Bragg peak is also related to the 'beam range', which is usually defined as the position where the dose is 80% of the value at the Bragg peak. Since the Bragg peak position depends on the energy of the particle beam, it is evident that by precisely controlling and modifying the energy, one can place the Bragg Peak at a given depth of a predetermined region, such as a tumor, so as to administer the greatest radiation energy to selected points and spare the healthy tissue surrounding said points.

The location of the Bragg peak within the tissue should be precisely known, since critical tissue localized near the target tumor could receive an excessive dose, whereas conversely the target tumor could receive an insufficient dose.

US 8,461,559 discloses a method for evaluating radiation model data in particle beam radiation applications, in particular in proton beam therapy of a determined target volume of malignant tissue within a patient, including the following steps: a) gaining diagnostic data for a determined target volume to be irradiated; b) calculating a particle range in the predetermined target volume based on the diagnostic data for the determined target volume; c) designing a radiation model with particle beam characteristics based on the calculated particle range and optionally on a calculated dose depth distribution; d) applying a single pencil beam shot to the determined target volume at an elevated beam energy as compared to the particle beam characteristics of the radiation model; e) measuring the beam range of the single pencil beam shot downstream of the determined target volume; and i) comparing the measured beam range to a reference beam range calculated on the basis of the radiation model.

"Patient-specific stopping power calibration for proton therapy planning based on single-detector proton radiography", P. J. Doolan, M. Testa, G. Sharp, E. H. Bentefour, G. Royle and H.-M. Lu, Physics in Medicine and Biology, Volume 60, Number 5, 2015, discloses an optimizer that can produce patient-specific calibration curves to convert x-ray computed tomography (CT) numbers to relative stopping powers (HU-RSPs) for proton therapy treatment planning. The difference between a digitally reconstructed radiograph water-equivalent path length (DRRWEPL) map through the x-ray CT dataset and a proton radiograph (set as the ground truth) is minimized by optimizing the HU-RSP calibration curve.

**Summary of the invention**

The present invention seeks to provide improved information that can be used to improve particle radiation treatment.

According to a first aspect of the present invention, an apparatus for aligning a volumetric dataset to particle beam radiography data is provided, comprising

a particle source for applying a particle beam directed toward an object, wherein the particle source is configured to scan at least part of the object with a plurality of pencil beam shots at a beam energy sufficiently high so as to allow the pencil beam to reach through and beyond the object, wherein the plurality of pencil beam shots are differently positioned with respect to the object;

a particle detector for measuring integral depth dose distributions of the respective pencil beam shots downstream of the object; and

a controller configured to:

　calculate information about a three-dimensional map of stopping power of the object based on a volumetric dataset representing the object,

　calculate reference depth dose distributions corresponding to the respective pencil beam shots based on the three-dimensional map of the stopping power and an assumed position of the object relative to the respective pencil beam shots,

　compare the measured depth dose distributions to their corresponding reference depth dose distributions, and

compute a difference between an actual position of the object and the assumed position of the object based on an outcome of the comparison.

The apparatus may thus find the actual position of the object with respect to each of the pencil beam shots in the volumetric dataset with a greater precision. Thus, the measured depth dose distributions can be mapped to the data of the volumetric dataset with a greater precision. This allows, for example, to control the particle source to irradiate a target region derived from the volumetric dataset with a greater precision. It also allows to position the object with greater precision in a particle radiation system before any therapeutic treatment with particles would be performed.

The controller may be configured to, after the controller has computed the position of the object, adjust the stopping powers of the three-dimensional map of stopping power based on at least one of the measured depth dose distributions. This way the accuracy of the three-dimensional map may be improved. For example, after the volumetric dataset has been aligned with the pencil beam shots with greater precision using the computed position of the object, the estimation of stopping power may be further improved by comparing the measured depth dose distribution to the (better-aligned) reference depth dose distribution again.

The controller may be configured to calculate the information about the three-dimensional map of stopping power based on a calibration curve mapping voxel values of the volumetric dataset to stopping power values, and adjust the calibration curve based on the computed position of the object relative to the pencil beam shots and at least one of the measured depth dose distributions. This provides a more accurate determination of the calibration curve, allowing to perform more accurate range estimation, which is an important parameter for treatment planning.

The controller may be configured to repeat the steps of calculating information about the three-dimensional map of stopping power, calculating the reference depth dose distributions, comparing the measured depth dose distributions, and computing the position of the object, based on the adjusted calibration curve. This may lead to an even more accurate determination of the position. Also, the step of adjusting the calibration curve may be repeated using the new position, to obtain even more accurate calibration curve. The whole process may be repeated until the process has converged.

The controller may be configured to, as part of the comparison, calculate a cost value based on differences between the measured integral depth dose distributions and the reference integral depth dose distributions; and, as part of the computation of the position of the object, find the position of the object associated with a reduced cost value by repeatedly calculating the reference depth dose distributions associated with adjusted assumed positions of the object and calculating the corresponding cost value. The calculation of the cost value allows the controller to adjust the assumed position in such a way that the cost function decreases, for example by trial and error, thereby obtaining an improvement in the accuracy of the assumed positions. The cost value may be indicative of how closely the measured integral depth dose distributions resemble the reference integral depth dose distributions.

The controller may be configured to perform the calculation of the cost value based on curve feature extraction, and to thereby operate a dimensionality reduction from each reference or measured integral depth dose to extract the cost value. This allows to efficiently determine the cost value using the available information in the integral depth dose distributions.

The controller may be configured to perform a depth shift of the reference integrated depth dose distribution with respect to the measured integrated depth dose distribution corresponding to a pencil beam shot, and to calculate the cost value based on the depth shift. This may lead to a more accurate determination of the position of the object.

The calculation of the reference depth dose distributions by the controller may comprise simulating the pencil beam shots through the object using at least one of the following methods: ray-tracing by casting a plurality of weighted rays; Gaussian kernel convolutions; cone-collapse; and Monte-Carlo simulations. These methods may lead to an accurate determination of the position of the object.

The plurality of pencil beam shots applied by the particle source may be transmitted in parallel or divergent directions with respect to the object. Moreover, the controller may be configured to combine the measured integral depth dose distributions of the respective pencil beam shots to create a particle radiograph of the object, and register the particle radiograph with the volumetric dataset representing the object based on the computed position. This allows to obtain a great accuracy throughout the object. The particle radiograph can be displayed as a picture of the object, for example. Also it allows to combine the information of the particle radiograph with the information of the volumetric dataset with great precision, for example in a combined visualisation.

The particle source may be configured to apply a plurality of sets of pencil beam shots, wherein the pencil beam shots of each set have a different direction with respect to the object than the pencil beam shots of the other sets. Moreover, the controller may be configured to combine the measured integral depth dose distributions of the respective pencil beam shots of each set of pencil beam shots to create a particle radiograph of the object corresponding to each set of pencil beam shots, and register the particle radiograph corresponding to each set of pencil beam shots with the volumetric dataset representing the object based on the computed position. This makes it easier to correct for rotations of the object around all three axes of rotation.

The controller may be configured to determine an affine transformation representing the computed position of the object relative to the pencil beam shots. An affine transformation, typically comprising rotations and/or translations, is highly suitable to cover most frequently occurring motion effects.

The volumetric dataset can comprise, for example, a CT (X-ray computed tomography) dataset or an MRI (magnetic resonance imaging) dataset.

The particle detector may comprise at least one of: a multi-layer ionization chamber (MLIC), a multi-layer Faraday-cup, multi-layer scintillating detectors, or a multi-layer stacked dose measurement system. Alternatively, any other particle detection system that is able to directly or indirectly reconstruct integral depth doses may be provided.

According to another aspect of the invention, a method of aligning a volumetric dataset to particle beam radiography data is provided, comprising:

gaining a volumetric dataset representing an object;
calculating information representing a three-dimensional map of stopping power of the object based on the volumetric dataset;
scanning at least part of the object with a plurality of pencil beam shots at a beam energy sufficiently high so as to allow the pencil beam to reach through and beyond the object, wherein the plurality of pencil beam shots are differently positioned with respect to the object;
measuring depth dose distributions of the respective pencil beam shots downstream of the object;
calculating reference depth dose distributions corresponding to the respective pencil beam shots based on the three-dimensional map of the stopping power and an assumed position of the object relative to the respective pencil beam shots;
comparing the measured depth dose distributions to their corresponding reference depth dose distributions; and
computing a difference between an actual position of the object and the assumed position of the object shots based on an outcome of the comparison.

The comparing may comprise calculating a cost value based on differences between the measured integral depth dose distributions and the reference integral depth dose distributions; and
the computing may comprise finding the position of the object associated with a reduced cost value by repeatedly calculating the reference depth dose distributions associated with an adjusted assumed position of object and calculating the corresponding cost value.

The person skilled in the art will understand that the features described above may be combined in any way deemed useful. Moreover, modifications and variations described in respect of the apparatus may likewise be applied to the method, and modifications and variations described in respect of the method may likewise be applied to the apparatus.

Brief description of drawings

The present invention will be discussed in more detail below, with reference to the attached drawings. Throughout the drawings, similar items have been indicated with the same reference numerals.

Fig. 1 illustrates an apparatus for aligning a volumetric dataset to a particle beam radiography data.
Fig. 2 illustrates a perspective view of an apparatus capable of generating and detecting particle radiation.
Fig. 3A shows a human head with an indication of a position of a pencil beam.
Fig. 3B shows a measured IDD for the pencil beam indicated in Fig. 3A.
Fig. 4A shows a phantom human head with a general indication of pencil beams.
Fig. 4B shows two graphs showing measured IDDs for the pencil beams indicated in Fig. 4A.
Fig. 5 illustrates a method for aligning a volumetric dataset to particle beam measurement data.
Fig. 6 shows a graph of a measured IDD and a graph of a simulated IDD.
Fig. 7A-F show graphs of a cost function versus optimization parameters.
Fig. 8 shows an example of a calibration curve.

Detailed description

According to the present disclosure, proton radiography (PR) using a multi-layer ionization chamber (MLIC) or any other device for measuring integral depth dose (IDD) can be used for patient positioning. Because IDDs carry information about lateral heterogeneities along the path of a particle beam, it is possible to reach submillimetre accuracy even with a spot spacing of 5mm. Although the present detailed disclosure describes the example of proton radiography in detail, the techniques may be applied to other kinds of particle radiation, such as alpha (helium ions), neon ions, oxygen ions, carbon ions, or any kind of particle radiation beam.

In the following, several terms and acronyms are defined and explained in the context of the present disclosure. The definitions refer to the terminology used in the context of medical imaging, radiotherapy, particle beam therapy, and in particular proton therapy. CT means computed tomography using X-ray data acquired by an X-ray CT scanner. SPR means stopping power ratio relative to water. HU means Hounsfield Units, which are units of the CT dataset, typically an indicator of the relative electron density. WET means Water Equivalent Thickness, which is the thickness of water that would provide the same integrated stopping power for protons (therefore the same loss of energy) as a certain sample of material. For example, a particular trajectory of a proton through an object has an associated water equivalent thickness. IDD means integral depth dose, which is a dose profile measured along the beam axis (or depth axis) of a proton beam. For each point of measurement, the dose is (physically) integrated in the plane lateral to the beam axis, hence the name 'integrated depth dose'. MLIC means Multi-layer ionization chamber, which is an apparatus allowing to measure an IDD by means of a stack of ionization chambers. PR means proton radiograph. A proton radiograph can be acquired, for example, using an MLIC in combination with spot scanning, which provides a 2D lateral resolution.

One of the limitations to fully exploit the ballistic advantages of protons in proton therapy is the uncertainty on the exact range inside an object, such as a patient. This uncertainty is mainly due to the lack of knowledge of the elemental composition of the tissues of the object. Indeed the treatment planning is based on classical CT, which gives information related to the electronic density of the tissues, to which the X-rays are sensitive. But the proton range is depends on another physical property, the stopping power, which depends not only on the density but also on the atomic composition of the matter. Unfortunately, X-ray detection can't properly distinguish different materials of similar densities, while protons are specifically sensitive to the stopping power. This uncertainty is accounted as up to 1.8% of the range among the 3.5% added as distal margin.

By performing imaging based on a beam of protons instead of X-rays, it is possible to provide more relevant information regarding the stopping power of the tissues within the object. However, this presents some difficulties. First, contrary to the X-rays, the protons don't follow a straight line through the matter, but trace sinuous paths due to the multiple coulomb scattering (MCS). Second, the protons cross thin detectors without delivering all their energies; the intensity response of a pixel of a simple 2D imager provides only information about the position of the proton, but not about the materials crossed by the proton.

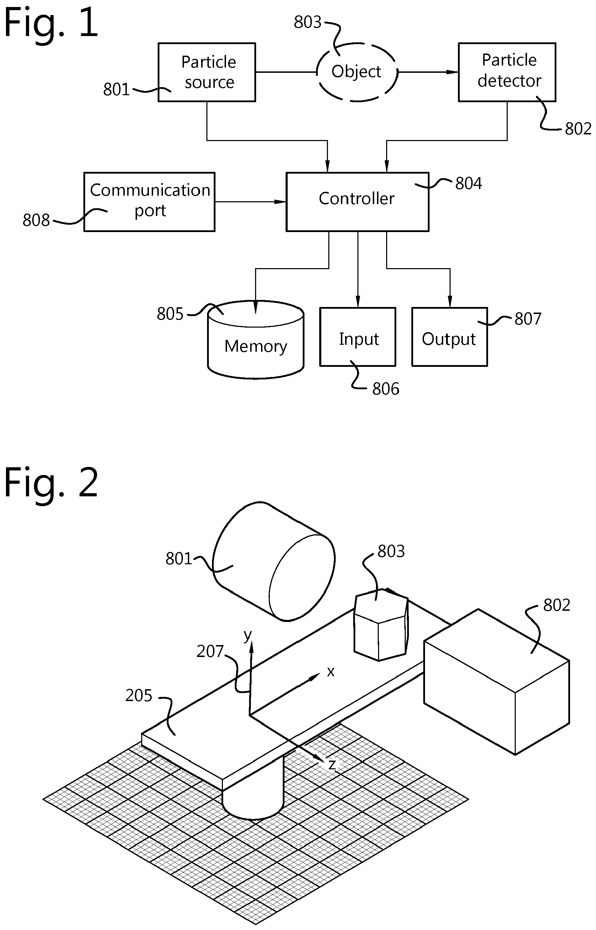

Fig. 1 shows a diagram of an apparatus for aligning a volumetric dataset to a particle beam radiography dataset. The apparatus comprises a particle source 101, a particle detector 102, and a space for an object 103 in between the particle source 101 and the particle detector 102. The apparatus further comprises a controller 804, which is configured to control operations of the apparatus. The controller 804 may be implemented as one or more computer processors, for example. The apparatus comprises a memory 805, which may comprise volatile and/or non-volatile memory. The memory 805 may comprise RAM, ROM, FLASH, and/or magnetic disc storage, for example. In the memory 805, a program may be stored that, when loaded by the controller 804, causes the controller to control the particle source 801, particle detector 802, and other components of the apparatus. The control unit 804 may also be configured, via the program stored in the memory 805, to perform computations and data transformations in order to register the measurement data generated by the particle detector on another volumetric dataset. The apparatus may comprise a communication port 808 to communicate with other devices, in order to receive datasets made with other radiography modalities, for example three-dimensional datasets representing objects, for example CT datasets or MRI datasets. These datasets may also be stored in the memory 805. The apparatus may further comprise an input 806, such as a user input device to receive commands from a user, and an output 807, such as a display and/or an audio speaker, for outputting data and signals to the user. The particle source 801 may comprise any generator of a beam of particles, as are known in the art by itself. For example, the particle source 801 may comprise a proton source that emits a beam of protons in the direction of the space for the object 803. The particle detector 802 is a detector for the particles that are emitted by the particle source 801. For example the particle detector comprises a proton detector. The detector 802 is configured to detect the location inside the detector where the particle or particles release their energy and the amount of energy released by the particle or particles that reach the detector.

The particle source 101 is configured to apply a particle beam directed to the space for the object 803. More particularly, the particle source 101 may be configured to transmit a pencil beam of particles. Such a pencil beam is a narrow beam, which may have a finite diameter. Typically, the radiation intensity within the pencil beam is the highest in the centre of the beam and lower near the periphery of the cross section of the beam. For example the two-dimensional cross sectional radiation intensity distribution of the pencil beam may be Gaussian. The particle source 801 may be configured to scan at least part of the object with a plurality of pencil beam shots at a beam energy sufficiently high so as to allow the pencil beam to reach through and beyond the object. The plurality of pencil beam shots are differently positioned with respect to the object. The pencil beam shots may be applied in sequence, and the object 803 may be moved in between the pencil beam shots, so that the pencil beam intersects different portions of the object 803. For example, the apparatus may comprise a support (205; illustrated in Fig. 2) on which the object may rest, and moving the object may be realized by moving the support. In a particular example implementation, the particle source 801 and/or the particle detector 802 may be moved and/or rotated. The particle detector 802 is configured to measure integral depth dose distributions of the respective pencil beam shots downstream of the object. Thus, after the particles have crossed the object, they are received by the particle detector 802 and release their energy inside the particle detector 802. The particle detector 802 is configured generate an integrated depth dose distribution. That is, the particle detector 802 detects where along the direction of the beam the energy is released by the particles. The energy in planes perpendicular to the direction of the beam is integrated by the particle detector 802. This way an integrated depth dose distribution is generated. The controller 804 is configured to receive a volumetric dataset, for example by means of the communication port 808. Alternatively, the volumetric dataset is retrieved from the memory 805. Yet alternatively, the apparatus comprises a volumetric imager, such as a CT scanner (not shown in the drawing), which generates the volumetric dataset by scanning the object. The controller 804 is configured to calculate information about a three-dimensional map of stopping power of the object based on a volumetric dataset representing the object. This may be done by using a transformation map or a calibration curve that maps the voxel values of the volumetric dataset to stopping power values. The transformation map or calibration curve may comprise a table that maps voxel values of the volumetric dataset to stopping power values. The voxel values may for example be CT values or, in case the volumetric dataset comprises an MRI dataset, T1 or T2 values. The stopping power may be expressed, for example, as a stopping power ratio that defines a ratio of the actual stopping power to the stopping power of water. The transformation map or calibration curve may be stored in the memory 805 or received via the communication port 808. How to create such a transformation map or calibration curve is, by itself, known to the person skilled in the art. Although the following description discloses detailed embodiments using a CT dataset with CT values (or Hounsfield units), the techniques may be applied similarly to other kinds of three-dimensional datasets with voxel values that are expressed in different units, such as MRI datasets with e.g. T1 or T2 values. Thus, features described herein involving the Hounsfield units may be modified to process the voxel values produced by the detection modality, such as T1 or T2 values.

The apparatus is configured to detect and store position information, i.e., the relative positions of the different pencil beams with respect to the object. That is, the distance and rotation values between the different pencil beams is recorded accurately. For example, the support may have an actuator that can move the support carrying the object 803 with great precision, for example using a stepper motor. Also, if the gantry of the apparatus is rotatable, the rotation angles of the particle source 801 and particle detector 802 in between the pencil beam shots are record. Thus, the main inaccuracy regarding the positioning of the pencil beam shots is due to the inaccuracy of the position of the object 803 (and possible shape deformations of the object itself). The controller 804 may be configured to receive information regarding the position of the object in the space for the object 803, and also information regarding the position of the object in the volumetric dataset. From this information, the controller 804 may compute an estimated intersection of each pencil beam with the volumetric dataset. Since the relative displacement between the pencil beams is accurately known, after the position of one pencil beam with respect to the volumetric dataset has been determined, the remaining pencil beams may be located with respect to the volumetric dataset based on this relative displacement. The controller 104 is configured to associate the appropriate calculated stopping power values of the three-dimensional map of stopping power to each pencil beam based on the determined position of each pencil beam. Using these associated calculated stopping power values, the controller 104 calculates reference depth dose distributions corresponding to the respective pencil beam shots. These reference depth dose distributions are thus computed from the volumetric dataset. In that sense the reference depth dose distributions are simulated depth dose distributions.

The controller is configured to compare the measured depth dose distributions to their corresponding reference depth dose distributions. This can be done in different ways, for example by calculating a cost value that represents the difference between the measured and reference depth dose distributions. The controller 104 is configured to compute the actual position of the object relative to the pencil beam shots based on an outcome of the comparison. To this end, the difference between the actual position of the object and the assumed position of the object may be computed. The controller 104 may carry out an optimization algorithm by altering the values for the positions of the pencil beams with respect to the volumetric dataset and re-computing the corresponding reference integrated depth dose distributions, and thereafter compare again the measured depth dose distributions to their corresponding reference depth dose distributions. Depending on whether the cost value increases or decreases, the values for the positions of the pencil beam shots may be further altered in the same direction or in another direction, or the process may be stopped if the comparison has reached an acceptable level.

The controller may be configured to calculate the cost value based on differences between the measured integral depth dose distributions and the reference integral depth dose distributions. These differences may be measured along the depth axis of the integral depth dose distributions and summed. The cost values for all the pencil beams may be summed to arrive at an overall cost value. The controller 104 may be configured to find the position of the object associated with a reduced cost value by repeatedly calculating the reference depth dose distributions associated with an adjusted assumed position of the object and calculating the corresponding cost value.

The controller 804 may be configured to perform the calculation of the cost value based on curve feature extraction, and to thereby operate a dimensionality reduction from each reference or measured integral depth dose to extract the cost value. The controller 804 may be configured to extract at least one feature from each reference integral depth dose or measured integral depth dose, and compute the cost value based on the extracted features.

The controller 804 may be configured to perform a depth shift of the reference integrated depth dose distribution with respect to the measured integrated depth dose distribution corresponding to a pencil beam shot. The controller may then calculate the cost value based on the determined depth shift.

The controller may be configured to, as part of the calculation of the reference depth dose distributions, simulate the pencil beam shots through the object using, for example, one or more of the following methods: ray-tracing by casting a plurality of weighted rays; Gaussian kernel convolution; cone-collapse, and Monte-Carlo simulations.

The particle source 801 and particle detector 802 may be configured to create a plurality of pencil beam shots that are transmitted in the same direction with respect to the object, by means of spot scanning. This may be done for example by moving the object in the space for the object 803 in between successive pencil beam shots. The controller 104 may be configured to combine the measured integral depth dose distributions of the respective pencil beam shots to create a particle radiograph of the object. For example, the particle radiograph may consist of the collection of the IDDs of the plurality of pencil beam shots. Alternatively, each IDD may be converted into a pixel value. Together, the pixels form a picture of the object. Such a conversion may be done by a conversion formula, some non-limiting examples of which are the maximum value of the IDD or the depth value of the peak of the IDD. The controller 104 may be configured to register the particle radiograph with the volumetric dataset representing the object based on the computed position.

The particle source 801 may be configured to apply a plurality of sets of pencil beam shots, wherein the pencil beam shots in each set correspond to a particle radiograph with a different orientation with respect to the object. Thus, the pencil beam shots of each set are transmitted by the particle source 801 in a different a different direction with respect to the object than the paths of the pencil beam shots of the other sets. This may be realized, for example, by moving the object 803 in between successive pencil beam shots. To generate sets with paths having different directions, the apparatus may be configured to rotate the object 803 in between the successive sets of pencil beam shots. In this case, the apparatus may comprise a rotatable robot arm that holds the object. Alternatively, the gantry (not shown) on which the particle source 801 and particle detector 802 are mounted may be rotated. The controller 804 may be configured to combine the measured integral depth dose distributions of the pencil beam shots of one set of pencil beam shots to create a particle radiograph of the object corresponding to that set of pencil beam shots. This may be performed for each set. The controller 804 may be configured to register the particle radiograph corresponding to each set of pencil beam shots with the volumetric dataset representing the object based on the computed position.

Further, the controller 804 may be configured to determine an affine transformation representing the computed position of the object relative to the pencil beam shots. In that case, the parameters to be optimized may include the elements of a matrix representing a linear transformation and a three-dimensional vector representing a translation. An affine transformation further allows to take deformations of the object into account in the registration.

The controller 804 may be configured to, after the controller has computed the position of the object, adjust the stopping powers of the three-dimensional map of stopping power based on at least one of the measured depth dose distributions. Based on the adjusted stopping powers and/or the determined actual position of the pencil beams with respect to the object, the controller 804 may adjust a particle radiation therapy plan based on the adjustment of the assumed position of the object, the adjusted stopping powers, and/or the adjusted calibration curve. This particle radiation therapy plan may be adjusted in a way known in the art by itself, by correcting the positioning of and the applied energy of the particles in a therapeutic particle beam emitted by the particle source 801.

The particle detector 802 may comprise at least one of a multi-layer ionization chamber (MLIC), a multi-layer Faraday-cup, multi-layer scintillating detectors, or a multi-layer stacked dose measurement system.

Fig. 2 shows an illustration of some of the components of a proton-radiography apparatus in a perspective view. The same reference numerals have been used in Fig. 1 and Fig. 2 to indicate similar components. The figure shows the particle source 801 and the particle detector 802, on opposite sides of an optional support 205. On the support, the object 803 to be measured may be rested. The drawing also shows a three-dimensional coordinate system 207, with x, y, and z axes. In the drawing, the z axis is generally parallel to an imaginary line connecting the center of the particle source 801 and the center of the particle detector 802. This z axis is also referred to as depth axis. The x axis and y axis are orthogonal to the z axis.

One of the possible aims of proton radiography is to improve the three-dimensional stopping power map, which is generally generated on the basis of a three-dimensional CT dataset. This conversion step is an important step in the proton therapy treatment planning workflow. This conversion from CT values (Hounsfield units) to stopping power values can be made by the following steps. First, a CT dataset is acquired and a proton radiograph is acquired. The expected IDD's of the proton radiograph are determined by simulation, using the CT dataset and a conversion curve that maps Hounsfield units to stopping power ratio. Thus, the Hounsfield values of the CT dataset are converted into stopping power values, and the IDD corresponding to a particular proton beam is computed by simulation. The actual acquired IDD and the simulated IDD are then compared. For example, the error in respect of water equivalent thickness (WET error) is determined as the difference between the water equivalent thickness computed from the acquired IDD and the water equivalent thickness corresponding to the simulated IDD. By optimizing the conversion from Hounsfield units to stopping power ratio, leading to smaller WET error, a more accurate conversion table between Hounsfield units and stopping power is obtained, so that a more accurate three-dimensional map of stopping power can be derived from the CT dataset.

The range error computation is very sensitive to alignment errors of the PR and the CT datasets. For example, a misalignment of 1mm would lead to important range errors, in particular on the regions having high heterogeneities, such as the outside boundaries of the object. Therefore, this misalignment represents an important confounding factor: while looking to range errors due to the CT-SPR map conversion curve, in fact range errors due to the patient alignment during the PR acquisition are measured.

The techniques disclosed herein may be used, among others, for at least two different purposes: First, the image registration may be carried out and used to separate the range errors caused by misalignment from range errors caused by imperfect conversion from CT to SPR. For example, first the alignment is improved, and then the conversion error may be determined using the correctly aligned data. Second, the techniques may be used to aid alignment of the object in a proton apparatus, using the CT image as a guide.

The present disclosure provides techniques to proceed to accurate "image" registration between PR and CT datasets. In a particular example, the method is based on a minimization of the residual error between PR acquisitions (acquired IDD's) and simulations made on the CT dataset (simulated IDD's). The said error can be computed in different ways. One example is the sum of the quadratic error between each simulated IDD and each measured IDD. The said simulations can be made, for example, using the following steps to generate a simulated IDD:

1. Convert the CT dataset into a three-dimensional SPR dataset.
2. Determine the WET of a path through the 3D SPR dataset (obtained by conversion from the CT dataset).
3. Shift of a reference IDD (at e.g. the highest energy) by the computed WET.
4. Weighting by a 2D Gaussian curve corresponding to the proton spot shape.

The above-mentioned steps will be explained in greater detail elsewhere in this document.

In a particular example, the registration itself can be done in 3 main steps, all of them being based on a minimization of the global WET error:

1. A two degrees of freedom registration (using translations perpendicular to the direction of the proton beam) (this step is optional).
2. A six degrees of freedom registration (using translation in all three directions and rotation around any of three orthogonal axes).
3. CT to SPR curve optimization, then iteration back to step 2.

It will be understood that variations in the degrees of freedom of the registration in each step are possible. For example, in step 2 the six degrees of freedom registration may be simplified as a five degrees of freedom registration using translation in two directions (for example the x and y axes shown in Fig. 2) and rotation around any of three orthogonal axes. The above-mentioned steps are described in further detail hereinafter.

A ray-tracing algorithm may be used to calculate simulated IDDs, for example to simulate the IDDs that were measured with the MLIC. The simulation takes into account the size of the beam and the fact that lateral heterogeneities along the path of the beam introduce range mixing. Such a model allows the development of a registration algorithm with submillimetre accuracy.

To perform the registration, a cost function can be defined to describe how much the IDD produced by simulation differs from the IDD provided by the MLIC. Then, the CT (converted to SPR) is moved with respect to the paths used to compute the simulated IDD and an interpolation method is used. A displacement of the CT provides a simulated IDD different than before, because the simulated path crosses a different portion of the CT/SPR dataset. The effect of a displacement is usually greater when lateral heterogeneities are present along the path of the beam. The fluence of a cross section of a pencil beam may be approximated by a two-dimensional Gaussian distribution. Herein, the fluence is the radiant energy received by a surface per unit area, integrated over the time of irradiation.

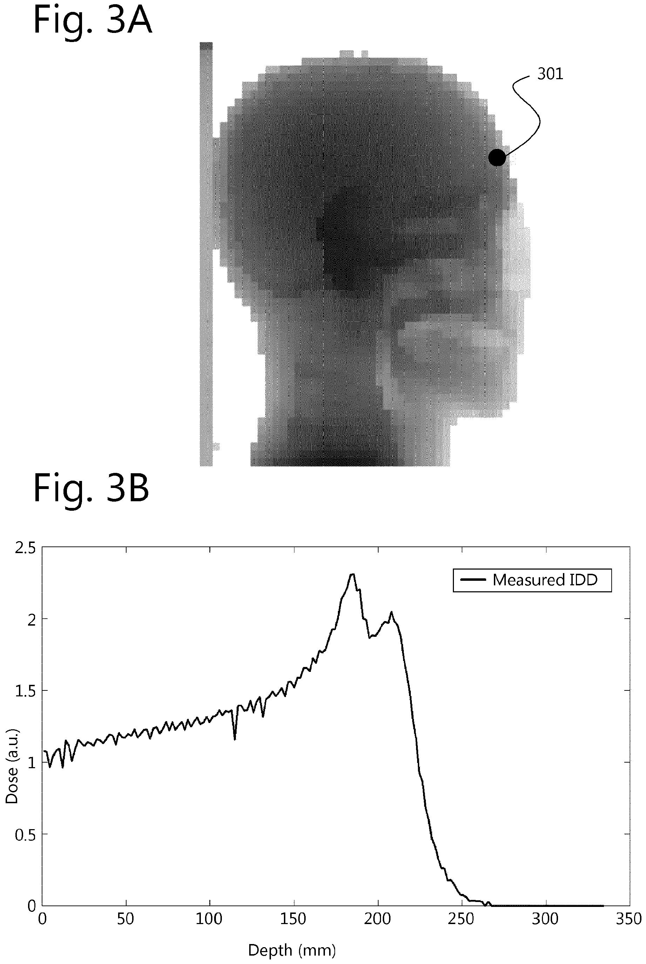

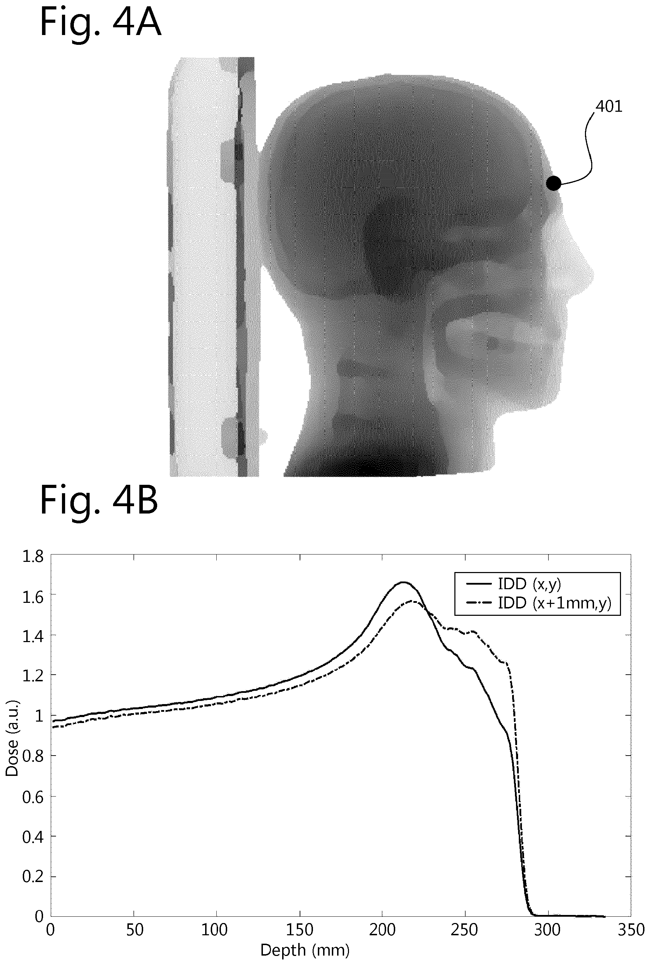

In a practical implementation, the standard deviation of that Gaussian distribution may be about 3 mm or larger. Because of that, the full width at half maximum (FWHM) may be greater than 8 mm. So an IDD does not only contain information about the WET at the centre of the pencil beam but also at the vicinity of this centre, as illustrated in Fig. 3. In Fig. 3A, a position of a pencil beam through a human head is indicated at numeral 301. As illustrated, the pencil beam crosses a boundary of the skull. Fig. 3B illustrates the measured IDD for that pencil beam, with on the horizontal axis the depth in mm and on the vertical axis the dose in arbitrary unit that the pencil beam emitted in the MLIC. The two peaks show that two sets of protons encountered different tissues on their way through the patient. Specifically, in this example a portion of the proton beam crossed the skull whereas the other portion of the proton beam only encountered soft tissue. In Fig. 4, the possible impact of a shift of the object with respect to the path of the beam is illustrated. Fig. 4A shows a phantom human head on a support and an indication 401 of a location where two pencil beams, with a lateral displacement of 1 mm, have been measured. Fig. 4B shows two graphs showing the IDDs for these two pencil beams, with on the horizontal axis the depth in mm and on the vertical axis the dose in arbitrary unit that the pencil beam emitted in the MLIC. It is shown that a remarkable difference is visible between these two IDDs despite the small displacement.

For example, a simulation may be performed based on the assumption that the pencil beams have a perfect Gaussian shape with constant sigma along all the path. Simulations using this assumption were shown to correspond very well to measurements, even in the case of lateral heterogeneities.

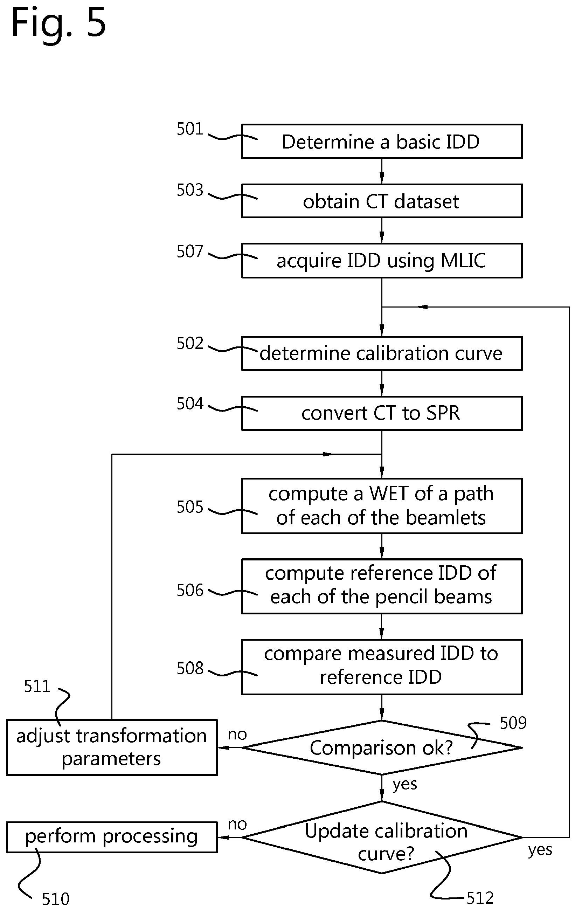

Fig. 5 illustrates a method for aligning a volumetric dataset to particle beam radiography data in greater detail.

In step 501, a 'basic IDD' is determined, corresponding to a known water equivalent thickness (WET), that could be any predetermined thickness, for example zero. For example, the basic IDD may be determined by means of a measurement of a proton beam that passes only air before it enters the MLIC, besides the components of the proton apparatus that are also used during the proton radiograph acquisitions. This basic IDD corresponding to a beam passing through air only is referred to herein as $IDD_{basic}$. The measurement may be performed at the manufacturing or configuration stage, and stored in a memory of the apparatus. Alternatively, the basic IDD may be measured shortly before the patient is placed in the measurement area.

In step 503, a volumetric dataset is obtained representing a particular object, for example using a CT scanner or an MRI scanner. In the following description, it will be assumed that a CT scanner is used, however this may be replaced by another type of volumetric scanner.

In step 507, the IDDs for which the reference IDD is computed in step 506, are measured by positioning the object in the proton apparatus, and causing the proton beam generator to send a beam of protons towards the object and the proton detector, such as the MLIC in the present example. The MLIC measures the amount of energy released by the protons, as well as the location (along the z-axis) where the energy is released. However, the MLIC integrates the energy of all beamlets in the x, y plane orthogonal to the z-axis, wherein the z-axis, or depth axis, is the axis parallel to the direction of the proton beam. Thus, the measured IDD is similar to the reference IDD as will be computed in step 506. It will be understood that the order of the steps can be changed; for example, the acquisition of the IDDs using the MLIC in step 507 is independent from the computation of the water equivalent thickness (WET) 505 and the computation of the reference IDDs 506. Also, it will be understood that steps 505, 506, and 507 can be performed for any desired number of beams and beamlets. Also, the directions of the beams need not be the same. In general, a set of measured IDDs for a set of parallel or diverging proton beams is called a proton radiograph. The proton beams of a proton radiograph may be organized to cover a particular area of interest (e.g. the whole object or a particular part of the object) by scanning the area using a predetermined spacing between the individual parallel proton beams. Multiple such proton radiographs may be acquired to view the object from different directions.

In step 502, a calibration curve is determined. This calibration curve may be retrieved from a memory of the apparatus, for example. The calibration curve may be determined by performing appropriate measurements during the manufacturing of the apparatus. How to determine such a calibration curve is known in the art by itself. The calibration curve maps values of a three-dimensional imaging modality, such as Hounsfield units of a CT dataset, into stopping power values, which may be expressed as stopping power ratios. Alternatively, values of a particular type of magnetic resonance data may be converted into stopping power values using a table. These conversion tables may be created as follows. First, it is determined what type of tissue corresponds to a particular value of a voxel of the volumetric dataset. Then, the stopping power of that type of tissue may be determined by experiment. The voxel value is then mapped to the stopping power of the corresponding tissue. If more tissue types have the same voxel value (e.g. the same Hounsfield value) but different stopping powers, a choice can be made for a particular stopping power, for example according to tissue types that are expected to be present in the object; this may lead to inaccuracies of the calibration table.

In step 504, the CT dataset is converted into stopping power values using the calibration curve of step 502. In certain embodiments, the stopping power values may be expressed as stopping power ratios, i.e. the ratio of the stopping power of the particular tissue and the stopping power of water.

In step 505, the stopping power encountered along the path of the beam is computed, by integration of stopping powers along the path. For example, the stopping power is computed as water equivalent thickness (WET), i.e. the integrated stopping power along the path of the beam is equivalent to the stopping power of a water column having a particular thickness. This thickness is a unit to express the stopping power.

The WET along the supposed path of the beam can be computed as follows, by combining the WET of a plurality of beamlets. Herein a beamlet is a mathematical entity corresponding to a trajectory that has a "zero thickness". Alternatively, a beamlet could represent a single particle (e.g. a proton). Assuming that all the beamlets that compose the beam are parallel to one another and the beam direction is parallel to a given axis z, and (x, y, z) forms an orthogonal coordinate system, the WET at position (x, y) in the plane can be computed using the following formula:

$$WET(x, y) = \sum_{k=1}^{N_Z} SPR(x, y, z_k) \, \text{spacing}(z)$$

Herein, spacing(z) denotes the size of the voxels in the z-direction, $N_z$ denotes the number of voxels in the z-direction, and $z_k$ denotes the z-values encountered along the beam.

In step 506, reference IDDs are computed by simulating the beam. This is based on the water equivalent thickness of each beamlet, that was computed in step 504, the basic IDD, determined in step 501, and the distribution of the density of the proton beam, which is represented by a two-dimensional Gaussian curve in the present example. Using these information, the IDD corresponding to a simulated beamlet for a position (x, y) would be as follows:

$$IDD(x, y, z) = IDD_{\text{basic}}(z + WET(x, y))$$

That is, a given WET of a beam path results in a shift of the IDD in the opposite direction of the beam, because for a larger WET, the proton would release its energy at a position within the MLIC closer to the surface of the MLIC facing the object and the proton source.

The simulated IDD, or reference IDD, denoted by $IDD_{direct}$ of a simulated beam can be a superposition of the IDDs of the beamlets that form the beam. Using the Gaussian distribution of the intensity of the particle beam, this superposition results in the following equation:

$$IDD_{direct}(x, y, z) = \sum_{i=1}^{N} G(x_i - x, y_i - y) IDD_{basic}(z + WET(x_i, y_i))$$

wherein G(x, y) is a two-dimensional Gaussian distribution representing the thickness and intensity distribution of the beam, wherein N denotes the number of beamlets that are computed to compute the reference IDD, and wherein ($x_i$, $y_i$), for $i$ = 1,2, ...,$N$ denote positions of the beamlets within the cross section of the beam.

In step 508, the measured IDDs are compared to the corresponding reference IDDs. This comparison may involve any kind of suitable metric. First, the IDDs of each measured beam may be compared to its corresponding simulated reference IDD. Then, the results of the comparison of the individual IDDs may be combined to arrive at an overall quality of the match between the entire set of proton beams and the CT dataset. For example, the sum of the quadratic error between a reference IDD and a measured IDD may be used to compare the measured IDD to the corresponding reference IDD. Alternatively, the range error in terms of the shift one can apply to a reference IDD to minimize the sum of the quadratic error between the reference IDD and the measured IDD can be used. Yet alternatively, the standard deviation of the absolute error between a reference IDD and a measured IDD can be used. Other examples are provided elsewhere in this description.

The overall cost value may be computed by adding the error values of the individual IDDs, for example. This summation may also be performed as a sum of squares. Also, the IDDs may be weighted differently in this summation step. For example, beams crossing a heterogeneous portion of the CT dataset may be given a higher weight than beams crossing a relatively homogeneous portion of the CT dataset.

In step 509 it is determined if the comparison of the IDDs is satisfactory. For example, it is determined if the overall cost value satisfies certain constraints, for example by checking if the overall cost value is below a predetermined threshold.

If the overall cost value does not yet satisfy the predetermined constraints in step 509, the transformation parameters are adjusted in step 511, according to an optimization algorithm, and then steps 505, 506, 508, and 509 are repeated with the adjusted transformation parameters.

In a particular implementation example, each parameter of a rigid registration is optimized one after another. These parameters include for example rotation around x-axis, rotation around y-axis, rotation around z-axis, translation in x direction, translation in y direction, and translation in z direction. Using such an approach can be advantageous from a viewpoint of computational expense. With such an approach, most of the steps do not need a transformation (such as a resampling) of the 3D-CT . For example, in certain implementations, in step 511 only one parameter is adjusted at a time, using an iterative optimization scheme. For example, one particular parameter will be adjusted for a predetermined number of iterations, or until a certain threshold on the cost value has been reached. In the iterations after that, the next parameter will be optimized.

The parameters are optimized using an iterative optimization scheme, that is: the IDDs are computed taking into account the changed transformation parameters, and the optimization scheme determines how the transformation parameters will be changed again, based on the outcome of the comparison between the corresponding computed reference IDD and the measured IDD. It will be understood that after changing the transformation parameters, the reference IDDs are computed again, but the measured IDD stays the same. No additional measurements need to be performed. Alternatively, a multivariate approach may be used for the optimization, which could lead to an even better accuracy, but could be slower than optimizing the parameters one by one.

If a plurality of proton radiographs are acquired, in different directions, then the different proton radiographs acquired in different directions may be used to optimize different transformation parameters. Herein, a proton radiograph is a collection of IDDs corresponding to a plurality of substantially parallel proton beams. For example, if two orthogonal directions are measured, one with proton beams parallel to a first axis and another one with proton beams parallel to a second axis, then the IDDs of the proton radiograph with proton beams parallel to the first axis may be used to optimize the rotation around the first axis, and to optimize the translation along two axes perpendicular to the first axis. The IDDs of the proton radiograph with proton beams parallel to the second axis may be used to optimize the rotation around the second axis, and translation along two axes perpendicular to the second axis. This still works even if the acquisition directions are not perpendicular.

To estimate the roll, i.e. the rotation around an axis that is orthogonal to both the first axis and the second axis, data from both proton radiographs can preferably be used. In principle, it is possible to use all proton radiograph data for optimizing each transformation parameter, however a computational efficiency is gained by using the above described selections of proton radiographs. This principle can be extended using more proton radiographs in more directions. In that case, more rotation and translation axes may be employed, which can thereafter be reduced to three rotation parameters and three translation parameters.

It is observed that, although in the above description, rigid registration parameters are described, the method also extends to more general transformations such as affine transformations or general non-rigid registration methods. The parameters of such transformations may be optimized one by one or using multivariate techniques.

After adjusting the transformation parameters in step 511, the method iterates the steps of 505, 506, 508, and 509, until the comparison value is found to be sufficient in step 509, after which the process proceeds to step 512.

In optional step 512, it is determined whether to update the calibration curve using the computed transformation parameters. If yes, the method proceeds to step 502 to determine the calibration curve using the computed transformation parameters. For example, after having determined the accurate position of the CT scan with respect to the pencil beams, the accurate mapping may be used to recalculate the calibration curve using the measured depth dose distributions and their adjusted assumed positions. To calculate or improve the calibration curve or the CT-to-SPR transformation based on measured IDDs, a method may be used as follows.

The 3D stopping power map of the patient may be adjusted based on the proton radiography data by producing a patient specific calibration curve. Once this curve is obtained, it can be used, for example, to convert the planning X-ray CT into an adjusted 3D SPR map.

Such a calibration curve may comprise a piecewise linear function that maps Hounsfield units (HU) to stopping power relative to water (SPR). In this approach, the HU and the SPR of different materials can be determined. For example, the following table shows the HU and the SPR for a few well known tissue types.

Table: HU versus SPR for different materials (see Woodard, H. Q., White, D. R., 1986, "The composition of body tissues", Br. J. Radiol. 59:1209-18)

| Material name | HU | SPR |
|---|---|---|
| Air | -1024 | 0 |
| Lung deflated | -741 | 0.26 |
| Adipose tissue 3 | -98 | 0.94 |
| Adipose tissue 1 | -55 | 0.98 |
| Soft tissue | 0 | 1 |
| Muscle skeletal 1 | 5 | 1 |
| Muscle skeletal 2 | 40 | 1.03 |
| Skin 1 | 72 | 1.05 |
| Connective tissue | 100 | 1.07 |
| Sternum | 385 | 1.18 |
| Humerus | 538 | 1.25 |
| Femur | 688 | 1.35 |

(continued)

| Material name | HU | SPR |
|---|---|---|
| Cranium | 999 | 1.53 |
| Cortical bone | 1524 | 1.82 |
| Titanium | 3200 | 3.19 |

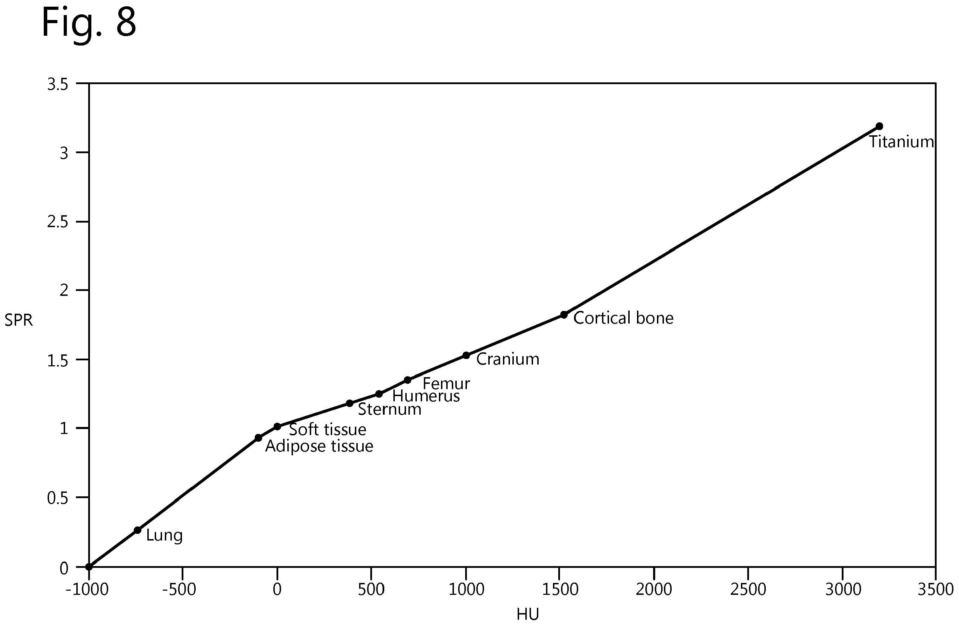

Table 1 shows a discrete relationship between HU and SPR for different materials. A continuous relationship can be obtained by assuming that the SPR of those materials are connected by linear segments (i.e. using linear interpolation). This results in a calibration curve such as the one shown illustratively in Fig. 8.

Using the measured IDD information, the calibration curve may be improved. This may be done in several ways. For example, an improved estimation of the SPR of the different materials may be calculated. From that information, the improved calibration curve may be determined using linear interpolation, for example. An improved estimation of the SPR of the different materials may be calculated, for example, by using the following equation:

$$x = \arg\min_{x}(\|Ax - b\|^2) \text{ , subject to } x \geq 0,$$

wherein x is a vector with dimension M, wherein M is the number of materials to be evaluated. The components of the vector x denote the SPR associated with each of the M distinct materials. Since the Hounsfield unit of those M materials is known, they can be used to update the above Table, and be used to improve the curve shown in Fig. 8. The materials to be used for this purpose may be chosen arbitrarily. For example, the materials represented in vector x may be chosen so as to correspond to typical human tissues, such as the ones listed in Table 1.

Moreover, A is an $N$ x M matrix that represents an estimation of the amount of interaction of N particle pencil beam shots with each of the M materials. This amount of interaction may be an indication of a length crossed in a material by a pencil beam shot. This may be estimated in different ways, based on e.g. the CT dataset and the trajectory of each pencil beam shot through the CT dataset (while applying e.g. the Gaussian weight function to take the intensity of the beam into account). For each voxel of the CT dataset, the material may be determined as the material with the HU closest to the HU of the voxel. Alternatively, the material of the voxel is determined as a mixture of, for example, two different materials, namely the material with the smallest HU that is larger than the HU of the voxel and the HU of the material with the largest HU that is smaller than the HU of the voxel. For each voxel and for each material the fraction of the voxel that is occupied by that material may be estimated. Then, the length $a_{ij}$ crossed in a material $j$ by a pencil beam $i$ can be computed as

$$a_{ij} = \sum_{\text{all voxels } v} ((\text{estimated length of pencil beam } i \text{ through voxel } v)$$
$$\times (\text{estimated fraction of the voxel } v \text{ that is occupied by material } j))$$

Further, b is an N-dimensional vector representing the WET values derived from N measured IDD of the N pencil beam shots. For instance, a table may be used that maps the range R-80 of an IDD to the corresponding WET value, taking into account the initial energy of the particles.

Referring to the equation $x = \arg\min(\|Ax - b\|^2)$, subject to $x \geq 0$, additional constraints on the variation of the stopping power of the materials may be set. For instance, equality constraints may be used for certain known materials, such as the materials corresponding to air and water. Also, the interpolation method to create the calibration curve may be varied. For example, the linear interpolation may be replaced by another kind of interpolation.

By adding more equality constraints, i.e. by imposing the SPR of certain materials to predefined values, is possible to optimize the SPR of only a few selected materials, or even only one single material. In particular, this may be useful when one specific material is known to have a considerable impact on the range uncertainty. A typical example is a metal implant. In such a case, for example, a single pencil beam may be used to optimize the SPR corresponding to this specific material, although several shots may increase the quality of the result.

Since on the one hand the quality of the optimized calibration curve might be impacted by set up errors and on the other hand the accuracy of the registration algorithm might be impacted by an incorrect calibration curve, the accuracy of both methods can be improved by combining them in an iterative process. For example, registration of proton radiography data followed by optimization of the calibration curve may be iteratively performed until no major change in the outputs of both methods are observed.

Once the calibration curve is finished, the SPR for each voxel may be determined by applying the calibration curve to the Hounsfield unit of the voxel. This way, an improved three-dimensional map of stopping power is obtained.

After the calibration curve has been updated in step 502, the CT-to-SPR conversion may be performed again in step 504 using the updated calibration curve. Then, either the method may end, and other operations may be performed as described for step 510, using the updated calibration curve and/or updated three-dimensional SPR map. Alternatively, the method proceeds by re-optimizing the positions, making use of the updated three-dimensional SPR map, in steps 505, 506, 508, 509, and 511.

If the cost value satisfies the predetermined constraints in step 509, and no update of the calibration curve is decided in step 512, the method proceeds to step 512, in which general operations are performed using the determined position of the object represented by the CT scan relative to the proton beams (that is, relative to the proton apparatus), and/or using the updated calibration curve or updated three-dimensional SPR map. For example, the information may be used to compute beam ranges to target a certain region of the object. Another example is a blended visualization of the CT dataset with the proton radiograph. Other uses area also possible.

The particle source may be configured to emit a pencil beam shot having a finite diameter, and therefore including a plurality of protons emitted in a narrow bundle. The depth dose distribution calculation of steps 505 and 506 disclosed above takes this into account by computing the reference IDD corresponding to a pencil beam as a weighted sum of a plurality of IDD's caused by individual protons or beamlets. The weights of the weighted sum may account for a beam intensity throughout the cross section of the pencil beam. This approach allows to take into account, among others, the fact that due to the finite size of the cross section of the pencil beam, multiple Bragg peaks may be detected arising from a single pencil beam shot.

The comparison of measured IDD with reference IDD, shown in step 508, may involve performing a curve feature extraction, which operates a dimensionality reduction of each reference or measured integral depth dose by extracting a single value or several values that describe the measured IDD and the reference IDD, respectively. These extracted features can thereafter be compared. For example, such feature extractors may be based on depth values, such as peak, R90, R80, center of mass, integral below curve, median, mean, variance, flatness, kurtosis or any statistical moment. Such features can be determined for both the measured IDD and the reference IDD, and then compared. This results in a comparison of the measured IDD and the reference IDD.

The comparison of the measured IDD with the corresponding reference IDD, shown in step 508, may also involve performing a depth shift of the measured IDD or the reference IDD relative to each other. The depth shift may be determined for which the comparison shows the most similarity, for example the depth shift corresponding to a lowest cost value found. As in other examples, the comparison may involve a sum of differences, sum of squared differences, variance, among others. The depth shift needed to obtain the greatest similarity between the curves can also, by itself, be used as a cost value indicative of the similarity of the measured IDD and the reference IDD.

The comparison illustrated in step 508 may also be preceded by a normalization step: first the reference IDD and the measured IDD may be normalized, so that the comparison provides more relevant results. The actual comparison, e.g. sum of squared differences or other, may be performed on the normalized IDDs.

The coordinate systems used for the computations and settings of the apparatus may be based on the definitions of IEC 61217 standard, for example. Symbols $R_x$, $R_y$, and $R_z$ are used in this disclosure to denote the three rotation matrices whose rotation axes are respectively x, y, and z. The associated rotation angles are named $\theta_x$, $\theta_y$, and $\theta_z$, The translations along the x-axis, y-axis, and z-axis are denoted by Symbols $T_x$, $T_y$, and $T_z$, respectively. The transformation may be applied according to the following convention:

$$\begin{bmatrix} x' \\ y' \\ z' \end{bmatrix} = R_y \, R_z \, R_x \begin{bmatrix} x \\ y \\ z \end{bmatrix} + \begin{bmatrix} T_x \\ T_y \\ T_z \end{bmatrix}$$

In other implementations, the transformations may be modified, for example the order in which the transformations are applied may be varied or the coordinate system may be changed, leading to different values of the transformation parameters. The transformation parameters may be summarized in a correction vector V, as follows:

$$V = \begin{bmatrix} \theta_x & \theta_y & \theta_z & T_x & T_y & T_z \end{bmatrix}$$

In order to fully benefit from all the information provided by proton radiography with a MLIC, the cost function can take into account range differences at the spot position, as well as range mixing due to the presence of lateral heterogeneities along the path of the beam. To this end, the use of an IDD simulation method may be incorporated inside the cost function evaluation. Such a simulation method, capable of accurately modelling range mixing, allows to make a high-quality comparison with the measured IDD.

In the following, several examples of suitable cost functions are disclosed, which may be used to determine the cost value used in the optimization process.

In the first example, the squared error between measured IDD called $IDD_{meas}$ and simulated IDD, named reference IDD or $IDD_{direct}$, by

$$SQE(x,y) = \int_0^{l_{detector}} (IDD_{direct}(x,y,z) - IDD_{meas}(x,y,z))^2 \; dz \quad,$$

wherein $l_{detector}$ denotes the length of the detector in the z-direction.

By summing this error over the available measured IDDs and by dividing by the number of spots (that is, the number of measured IDDs), a cost function is obtained, named $CF$, that represents the mean error to minimize:

$$CF = \frac{\sum_{x_i,y_i} SQE(x_i, y_i)}{N_{IDD}} \quad,$$

wherein $N_{IDD}$ denotes the number of measured IDDs and the summation $\sum_{x_i,y_i} SQE(x_i,y_i)$ denotes a summation of the SQE for all pairs $(x_i, y_i)$, corresponding to the measurement spots of the measured IDDs, with $i = 1,2, ..., N_{IDD}$.

Alternatively, instead of the squared error SQE disclosed above, the standard deviation of the difference between the simulation and the measurement may be used:

$$STD(x,y) = std(IDD_{direct}(x,y,z) - IDD_{meas}(x,y,z))$$

wherein the standard deviation is computed of the values of the subtraction for the values of z for which the measurement is available. Typically these values of z are in the range from 0 to $l_{detector}$.

By summing STD(x, y) for the available values of (x, y) and by dividing by the number of spots, a cost function is obtained, named $CF$, that represents the value to minimize:

$$CF = \frac{\sum_{x_i,y_i} STD(x_i, y_i)}{N_{IDD}} \quad,$$

wherein $N_{IDD}$ denotes the number of measured IDDs and the summation $\sum_{x_i,y_i} STD(x_i,y_i)$ denotes a summation of the STD for all pairs $(x_i, y_i)$, with $i = 1,2, ..., N_{IDD}$.

The range error map between simulated IDD and measured ones is very sensitive to misalignments. Hence, mean range error may be used to define an appropriate cost function. Because of range mixing and the resulting complex shape of IDD, there are different possible approaches to determining this range error. The range error is the difference between the expected range or ranges according to the reference IDD and the actual range or ranges as represented by (e.g. the peak or peaks of) the measured IDD. This difference, named *RD* hereinafter, may be evaluated for example by computing the range shift s that minimizes the squared error between the simulated curve and the measured curve:

$$RD(x,y) = \arg\min_{s} \int_{0}^{l_{detector}} (IDD_{direct}(x,y,z+s) - IDD_{meas}(x,y,z))^2 \, dz$$

By summing this error RD(x, y) over the available measured IDDs and by dividing by the number of spots (that is, the number of measured IDDs), a cost function is obtained, named *CF,* that represents the mean error to minimize:

$$CF = \frac{\sum_{x_i,y_i} RD(x_i, y_i)}{N_{IDD}},$$

wherein $N_{IDD}$ denotes the number of measured IDDs and the summation $\sum_{x_i,y_i} RD(x_i, y_i)$ denotes a summation of RD for all pairs ($x_i$, $y_i$), with $i$ = 1,2, ..., $N_{IDD}$.

Another example cost function only considers one value for each Bragg curve or IDD: the position of the maximum dose in the IDD:

$$MAX_{direct}(x,y) = \arg\max_{z} IDD_{direct}(x,y,z)$$

and

$$MAX_{meas}(x,y) = \arg\max_{z} IDD_{meas}(x,y,z)$$

Different cost functions can be defined based on $MAX_{direct}(x,y)$ and $MAX_{meas}(x,y)$. For example, a cost function can be based on the squared difference of those values:

$$CF = \frac{\sum_{x_i,y_i} (MAX_{direct}(x_i,y_i) - MAX_{meas}(x_i,y_i))^2}{N_{IDD}}$$

wherein $N_{IDD}$ denotes the number of measured IDDs and the summation $\sum_{x_i,y_i}$ denotes a summation for all pairs ($x_i$, $y_i$), with $i$ = 1,2, ..., $N_{IDD}$.

Another example cost function is based on the mean squared error after correction for the range error, as for example in the following definition of SQE2:

$$SQE2(x,y) = \int_{0}^{l_{detector}} (IDD_{direct}(x,y,z+RD(x,y)) - IDD_{meas}(x,y,z))^2 \, dz$$

This would lead to the following cost function:

$$CF = \frac{\sum_{x_i, y_i} SQE2(x_i, y_i)}{N_{IDD}}$$
,

wherein $N_{IDD}$ denotes the number of measured IDDs and the summation $\Sigma_{x_i, y_i} SQE2(x_i, y_i)$ denotes a summation of SQE2 for all pairs $(x_i, y_i)$, with $i = 1, 2, ..., N_{IDD}$.

Instead of the mean squared error, one could also consider the variance of the difference between the reference IDD and the measured IDD after correction for the range error, as for example in the following definition ofVAR:

$$VAR(x, y) = Var\left(IDD_{direct}(x, y, z + RD(x, y)) - IDD_{meas}(x, y, z)\right)$$

wherein the variance is computed of the values of the subtraction for the values of z for which the measurement data is available. Typically these values of z are in the range from 0 to $l_{detector}$. Instead of variance, the standard deviation may be used. This would lead to the following cost function:

$$CF = \frac{\sum_{x_i, y_i} VAR(x_i, y_i)}{N_{IDD}}$$
,

wherein $N_{IDD}$ denotes the number of measured IDDs and the summation $\Sigma_{x_i, y_i} VAR(x_i, y_i)$ denotes a summation of VAR for all pairs $(x_i, y_i)$, with $i = 1, 2, ..., N_{IDD}$.

One of several types of optimizers can be used, particularly in steps 509, 511. For example, a method known as 'coordinate descend', or a variation thereof, may be used. Such a method benefits from reduced computational time in the case of parallel beamlets and analytical simulations since geometrical transforms can be performed directly on the (projected) 2D WET-map instead of on the 3D volume. With this method, each one of the six parameters (the three rotation angles and the three translations) is determined in a sequential manner by a minimization algorithm which in this case is the golden section method, for example. Other optimization methods can also be used, such as a gradient descent method, for instance.

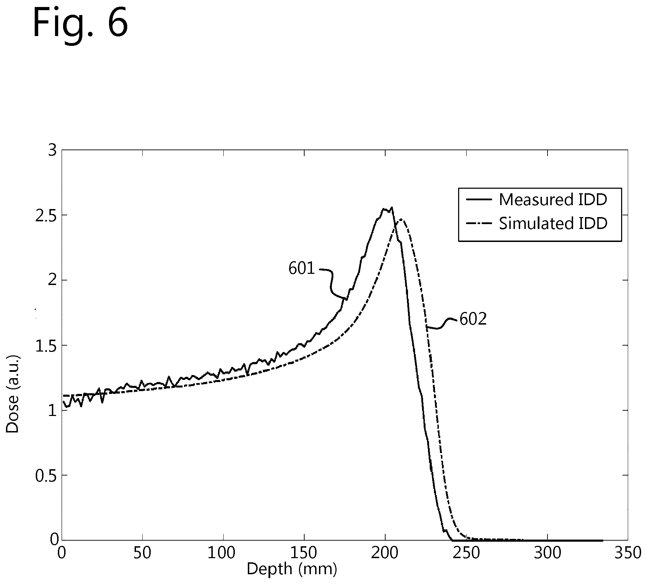

Fig. 6 shows, as an example, a graph of a measured IDD 601 of a proton beam through a head phantom, and a graph of a simulated, reference IDD 602 based on the same path through the same head phantom. The reference IDD was computed, in this case, based on a CT scan of the head phantom that was converted to a three-dimensional map of stopping power ratios (SPR). The measured IDD was measured using a proton source and a detector comprising an MLIC. In Fig. 6, the horizontal axis represents the 'depth' axis in mm, that is, the distance along the 'z' axis. The vertical axis denotes the detected energy, in an arbitrary unit. The measured data in this experiment was acquired on a head phantom (CIRS, USA) using a Giraffe (IBA Dosimetry, Belgium). The beam energy was 210 MeV and the spot spacing was 3mm. On the other hand, simulated data were obtained according to the equation for $IDD_{direct}$, as disclosed above:

$$IDD_{direct}(x, y, z) = \sum_{i=1}^{N} G(x_i - x, y_i - y) IDD_{basic}(z + WET(x_i, y_i))$$

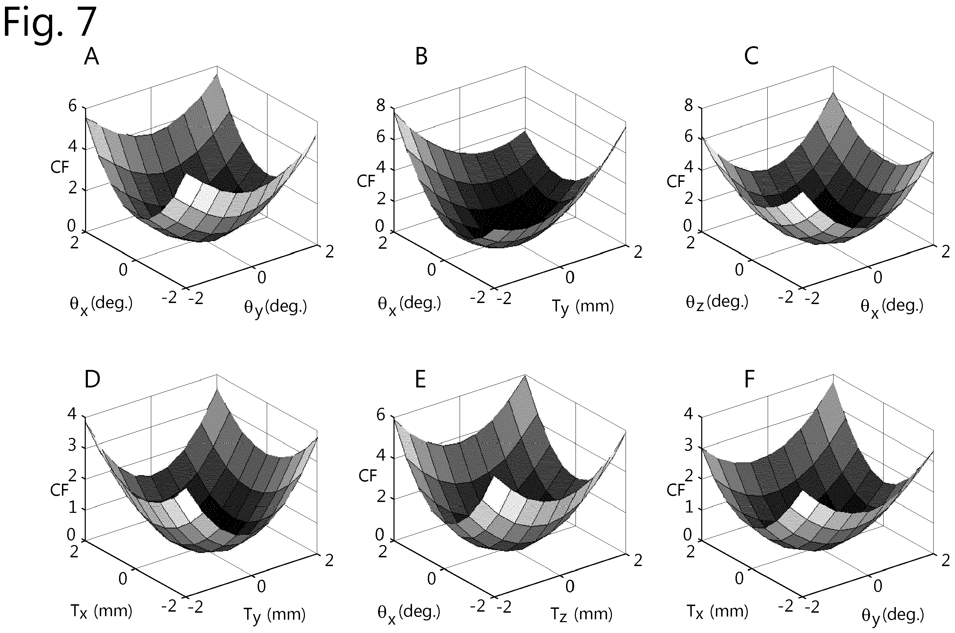

Fig. 7 shows an example of how the cost function varies with the optimization parameters $\theta_x$, $\theta_y$, $\theta_z$, $T_x$, $T_y$, and $T_z$. The shape of the cost function with respect to the six parameters (three angles and three translations) determines how well a given algorithm converges towards the optimal solution. Fig. 7 shows the value computed by the cost function:

$$CF = \frac{\sum_{x_i, y_i} SQE(x_i, y_i)}{N_{IDD}}$$

,

for rotation angles ranging from -2 to 2 degrees and translations ranging from -5 to 5 mm. Only six graphs are shown, showing different combinations of the six transformation parameters on the two horizontal axes and the value of the cost function CF on the vertical axis. The graphs for other pairs of transformation parameters are assumed to have similar shapes. For instance, the shape of the graph of the cost function with respect to rotations around x and y axis (Fig. 7A) is similar to the shape of the graph of the cost function with respect to translation along x axis and rotation around y axis (Fig. 7F). The apparent convex shape of the function allows the use of various well-known optimization tools. It is noted that the shape of the cost function depends on many factors, including the anatomy of the patient.

Some aspects of the invention may be suitable for being implemented in form of software, in particular a computer program product. The computer program product may comprise a computer program stored on a non-transitory computer-readable media. Also, the computer program may be represented by a signal, such as an optic signal or an electro-magnetic signal, carried by a transmission medium such as an optic fiber cable or the air. The computer program may partly or entirely have the form of source code, object code, or pseudo code, suitable for being executed by a computer system. For example, the code may be executable by one or more controllers or processors.

The examples and embodiments described herein serve to illustrate rather than limit the invention. The person skilled in the art will be able to design alternative embodiments without departing from the spirit and scope of the present disclosure, as defined by the appended claims and their equivalents. Reference signs placed in parentheses in the claims shall not be interpreted to limit the scope of the claims. Items described as separate entities in the claims or the description may be implemented as a single hardware or software item combining the features of the items described.

The present invention has been described above with reference to a number of exemplary embodiments as shown in the drawings. Modifications and alternative implementations of some parts or elements are possible, and are included in the scope of protection as defined in the appended claims.

**Claims**

1. An apparatus for aligning a volumetric dataset to a particle beam radiography data, comprising
a particle source (101) for applying a particle beam directed toward an object, wherein the particle source is configured to scan at least part of the object with a plurality of pencil beam shots at a beam energy sufficiently high so as to allow the pencil beam to reach through and beyond the object, wherein the plurality of pencil beam shots are differently positioned with respect to the object;
a particle detector (102) for measuring integral depth dose distributions of the respective pencil beam shots downstream of the object; and
a controller (104) configured to:

   calculate information about a three-dimensional map of stopping power of the object based on a volumetric dataset representing the object,
   calculate reference depth dose distributions corresponding to the respective pencil beam shots based on the three-dimensional map of the stopping power and an assumed position of the object relative to the respective pencil beam shots,
   compare the measured depth dose distributions to their corresponding reference depth dose distributions, and
   compute a difference between an actual position of the object and the assumed position of the object based on an outcome of the comparison.

2. The apparatus of claim 1, wherein the controller (804) is configured to, after the controller has computed the position of the object, adjust the stopping powers of the three-dimensional map of stopping power based on at least one of the measured depth dose distributions.

3. The apparatus of claim 1, wherein the controller (804) is configured to calculate the information about the three-dimensional map of stopping power based on a calibration curve mapping voxel values of the volumetric dataset to stopping power values, and wherein the controller (804) is configured to adjust the calibration curve based on the computed position of the object relative to the pencil beam shots and at least one of the measured depth dose distributions.

4. The apparatus of claim 3, wherein the controller (804) is configured to repeat the steps of calculating information about the three-dimensional map of stopping power, calculating the reference depth dose distributions, comparing the measured depth dose distributions, and computing the position of the object, based on the adjusted calibration curve.

5. The apparatus of claim 1, wherein the controller (104) is configured to:

as part of the comparison, calculate a cost value based on differences between the measured integral depth dose distributions and the reference integral depth dose distributions; and
as part of the computation of the position of the object, find the position of the object associated with a reduced cost value by repeatedly calculating the reference depth dose distributions associated with an adjusted assumed position of the object and calculating the corresponding cost value.

6. The apparatus of claim 5, wherein the controller (104) is configured to extract at least one feature from each reference integral depth dose or measured integral depth dose, and compute the cost value based on said at least one extracted feature.

7. The apparatus of claim 5, wherein the controller (104) is configured to perform a depth shift of the reference integrated depth dose distribution with respect to the measured integrated depth dose distribution corresponding to a pencil beam shot, and to calculate the cost value based on the depth shift.

8. The apparatus of claim 1, wherein the controller (104) is configured to, as part of the calculation of the reference depth dose distributions, simulate the pencil beam shots through the object using at least one of the following methods: ray-tracing by casting a plurality of weighted rays; Gaussian kernel convolution; cone-collapse; and Monte-Carlo simulations.

9. The apparatus of claim 1, wherein the plurality of pencil beam shots define parallel or divergent paths through the object, and the controller (104) is configured to
combine the measured integral depth dose distributions of the respective pencil beam shots to create a particle radiograph of the object, and
register the particle radiograph with the volumetric dataset representing the object based on the computed position.

10. The apparatus of claim 1, wherein the particle source (801) is configured to apply a plurality of sets of pencil beam shots, wherein the pencil beam shots of each set define paths that have a different direction with respect to the object than the paths of the pencil beam shots of the other sets, and the controller (804) is configured to:

combine the measured integral depth dose distributions of the respective pencil beam shots of each set of pencil beam shots to create a particle radiograph of the object corresponding to each set of pencil beam shots, and
register the particle radiograph corresponding to each set of pencil beam shots with the volumetric dataset representing the object based on the computed position.

11. The apparatus of claim 1, wherein the controller (804) is configured to determine an affine transformation representing the computed position of the object relative to the pencil beam shots.

12. The apparatus of claim 1, wherein the volumetric dataset comprises a CT dataset or an MRI dataset.

13. The apparatus of claim 1, wherein the particle detector (802) comprises at least one of: a multi-layer ionization chamber (MLIC), a multi-layer Faraday-cup, multi-layer scintillating detectors, or a multi-layer stacked dose measurement system.

14. A method of aligning a volumetric dataset to particle beam radiography data, comprising
gaining a volumetric dataset representing an object (503);
calculating information representing a three-dimensional map of stopping power of the object based on the volumetric dataset (504);
scanning at least part of the object with a plurality of pencil beam shots at a beam energy sufficiently high so as to allow the pencil beam to reach through and beyond the object, wherein the plurality of pencil beam shots are differently positioned with respect to the object, and measuring depth dose distributions of the respective pencil beam shots downstream of the object (507);

calculating reference depth dose distributions corresponding to the respective pencil beam shots based on the three-dimensional map of the stopping power and an assumed position of the object relative to the respective pencil beam shots (506);

comparing the measured depth dose distributions to their corresponding reference depth dose distributions (508); and computing a difference between an actual position of the object and the assumed position of the object based on an outcome of the comparison (509, 511).

# Fig. 1

```
                                803
┌──────────┐         ┌─────────┐         ┌──────────┐
│ Particle │         │  Object │         │ Particle │
│  source  │────────▶│         │────────▶│ detector │
└──────────┘         └─────────┘         └──────────┘
  801                                         802
     │                                         │
     │          ┌──────────┐                   │
     └─────────▶│Controller│◀──────────────────┘
┌──────────┐    │          │ 804
│Communica-│───▶│          │
│tion port │    └──────────┘
└──────────┘      │   │   │
  808             │   │   │
         ┌────────┘   │   └────────┐
         ▼            ▼            ▼
    ┌─────────┐  ┌────────┐  ┌────────┐
805 │ Memory  │  │ Input  │  │ Output │ 807
    └─────────┘  └────────┘  └────────┘
                     806
```

# Fig. 2

## Fig. 3A

## Fig. 3B

## Fig. 4A

401

## Fig. 4B

# Fig. 5

501 — Determine a basic IDD

503 — obtain CT dataset

507 — acquire IDD using MLIC

502 — determine calibration curve

504 — convert CT to SPR

505 — compute a WET of a path of each of the beamlets

506 — compute reference IDD of each of the pencil beams

508 — compare measured IDD to reference IDD

511 — adjust transformation parameters

no — Comparison ok? — 509

yes

perform processing — no — Update calibration curve? — yes

510

512

# Fig. 6

Fig. 7

Fig. 8

Europäisches
Patentamt

European
Patent Office

Office européen
des brevets

# EUROPEAN SEARCH REPORT

Application Number

EP 16 20 6519

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | FARACE PAOLO ET AL: "Technical Note: A direct ray-tracing method to compute integral depth dose in pencil beam proton radiography with a multilayer ionization chamber", MEDICAL PHYSICS, AIP, MELVILLE, NY, US, vol. 43, no. 12, 9 November 2016 (2016-11-09), pages 6405-6412, XP012213516, ISSN: 0094-2405, DOI: 10.1118/1.4966703 [retrieved on 2016-11-09] * the whole document * | 1,5-14 | INV. A61N5/10 |
| X,D | US 8 461 559 B2 (LOMAX ANTONY [CH]) 11 June 2013 (2013-06-11) * abstract * * column 3, lines 33-43 * * column 4, lines 12-18 * * column 5, line 6 - column 7, line 13 * * figures 1,7 * | 1-4, 12-14 | |
| X | PAOLO FARACE ET AL: "Pencil beam proton radiography using a multilayer ionization chamber", PHYSICS IN MEDICINE AND BIOLOGY, INSTITUTE OF PHYSICS PUBLISHING, BRISTOL GB, vol. 61, no. 11, 10 May 2016 (2016-05-10), pages 4078-4087, XP020305205, ISSN: 0031-9155, DOI: 10.1088/0031-9155/61/11/4078 [retrieved on 2016-05-10] * the whole document * | 1,5-7,9, 12-14 | TECHNICAL FIELDS SEARCHED (IPC) A61N |

-/--

The present search report has been drawn up for all claims

| Place of search | Date of completion of the search | Examiner |
|---|---|---|
| Munich | 6 June 2017 | Grochol, Jana |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

page 1 of 2

Europäisches
Patentamt

European
Patent Office

Office européen
des brevets

## EUROPEAN SEARCH REPORT

Application Number

EP 16 20 6519

### DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A,D | DOOLAN P J ET AL: "Patient-specific stopping power calibration for proton therapy planning based on single-detector proton radiography", PHYSICS IN MEDICINE AND BIOLOGY, INSTITUTE OF PHYSICS PUBLISHING, BRISTOL GB, vol. 60, no. 5, 10 February 2015 (2015-02-10), pages 1901-1917, XP020281343, ISSN: 0031-9155, DOI: 10.1088/0031-9155/60/5/1901 [retrieved on 2015-02-10] * abstract * | 1-14 | |
| A | SCHNEIDER UWE ET AL: "Patient specific optimization of the relation between CT-Hounsfield units and proton stopping power with proton radiography", MEDICAL PHYSICS, AIP, MELVILLE, NY, US, vol. 32, no. 1, 30 December 2004 (2004-12-30), pages 195-199, XP012075136, ISSN: 0094-2405, DOI: 10.1118/1.1833041 * abstract * | 1-14 | |

TECHNICAL FIELDS
SEARCHED      (IPC)

The present search report has been drawn up for all claims

| Place of search | Date of completion of the search | Examiner |
|---|---|---|
| Munich | 6 June 2017 | Grochol, Jana |

CATEGORY OF CITED DOCUMENTS

X : particularly relevant if taken alone
Y : particularly relevant if combined with another
    document of the same category
A : technological background
O : non-written disclosure
P : intermediate document

T : theory or principle underlying the invention
E : earlier patent document, but published on, or
    after the filing date
D : document cited in the application
L : document cited for other reasons

& : member of the same patent family, corresponding
    document

EPO FORM 1503 03.82 (P04C01)

**ANNEX TO THE EUROPEAN SEARCH REPORT
ON EUROPEAN PATENT APPLICATION NO.**                    EP 16 20 6519

This annex lists the patent family members relating to the patent documents cited in the above-mentioned European search report.
The members are as contained in the European Patent Office EDP file on
The European Patent Office is in no way liable for these particulars which are merely given for the purpose of information.

06-06-2017

| Patent document cited in search report | | Publication date | Patent family member(s) | | Publication date |
|---|---|---|---|---|---|
| US 8461559 | B2 | 11-06-2013 | CN | 102438700 A | 02-05-2012 |
| | | | EP | 2229981 A1 | 22-09-2010 |
| | | | EP | 2408523 A1 | 25-01-2012 |
| | | | JP | 5528533 B2 | 25-06-2014 |
| | | | JP | 2012520703 A | 10-09-2012 |
| | | | US | 2012056109 A1 | 08-03-2012 |
| | | | WO | 2010105858 A1 | 23-09-2010 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 8461559 B

**Non-patent literature cited in the description**

- **P. J. DOOLAN ; M. TESTA ; G. SHARP ; E. H. BENTEFOUR ; G. ROYLE ; H.-M. LU.** Patient-specific stopping power calibration for proton therapy planning based on single-detector proton radiography. *Physics in Medicine and Biology,* 2015, vol. 60 (5